# EUROPEAN PATENT APPLICATION

(11) **EP 1 475 084 A1**
(43) Date of publication of application: **10.11.2004**
(21) Application number: 03705117.4
(22) Date of filing: 13.02.2003
(51) Int. Cl.: A61K 9/20, A61K 47/32, A61K 47/36, A61K 47/38

(54) **TABLETS HAVING IMPROVED TABLETTING CHARACTERISTICS AND PROCESS FOR PRODUCING THE SAME**

(30) Priority: 15.02.2002 JP 2002038014
(71) Applicant: OTSUKA PHARMACEUTICAL CO., LTD., Chiyoda-ku Tokyo 101-8535 (JP)
(72) Inventor: KAWASAKI, Junichi, Itano-gun, Tokushima 771-0204 (JP)
(74) Representative: HOFFMANN - EITLE
(86) International application number: PCT/JP2003/001494
(87) International publication number: WO 2003/068194

(57) **Abstract**

It is intended to provide tablets which have a favorable texture in taking and excellent compressing characteristics, friability and mechanical strength. Tablets which are produced by blending a powdery or granular mixture comprising (1) an effective amount of a pharmacologically active substance and (2) 50% by weight or more of a water-soluble filler based on the powdery or granular mixture with (3) a compressing characteristic improving agent containing moisture at least in an amount corresponding to the equilibrated moisture content at 25°C under a humidity of 12% and having an average particle size of 100 µm or less, at a ratio of from 1 to 50% by weight based on the above mixture, and then compressing the resultant mixture.

## Description

### TECHNICAL FIELD

This invention relates to tablets having improved compressing characteristics and a process for producing the same, in particular, to orally disintegrating tablets and a process for producing the same.

### BACKGROUND ART

Orally disintegrating tablets are drug formulations that disintegrate rapidly in the oral cavity, and thus can be easily taken by those who are difficult to swallow ordinary tablets, such as pediatric patients and geriatric patients. Orally disintegrating tablets are desired to have an excellent mouth feel, such as acceptable taste and less rough texture after disintegration, because they disintegrate in the oral cavity. Thus water-soluble fillers, such as sugar and sugar alcohol, are blended into disintegrating tablets in a large proportion so as to improve the mouth feel thereof.

For example, WO 93/12769 and WO 94/12142 disclose orally disintegrating tablets which are produced by filling a solution or suspension of a pharmacologically active ingredient into a forming pocket and then freeze drying, air blast drying or vacuum drying the same. This type of orally disintegrating tablets is, however, poor in mechanical strength and the chipping off of the disintegrating tablets is unavoidable when removing the same from a PTP(push through package).

JP-A-5-271054 discloses orally disintegrating tablets which are produced by compressing a mixture including a pharmacologically active ingredient, saccharides to moisten the surface of the succharide grains. In the production of this type of disintegrating tablets, the moisture content of the mixture is adjusted before compressing by adding water to the surface of the saccharide grains. However, it is very difficult to adjust the mixture before compressing to have a uniform moisture content throughout. Furthermore, this production process involves filling a wet mixture, whose flowability is extremely low, into a tablet machine; as a result, it is unavoidable that the wet mixture sticks to and remains in the inside of the tablet machine, and besides when filling the wet mixture into a tablet machine, variations are created in the amount of the wet mixture filled into the machine. Still further, the production process described in JP-A-5-271054 requires a step of drying the resultant tablet after compressing.

WO 95/20380 discloses disintegrating tablets which include both a sugar alcohol low in compressibility and a sugar alcohol high in compressibility. This production process, however, has disadvantages in that increased content of sugar alcohol causes compressing defects such as capping. Thus the disintegrating tablets described in WO95/20380 do not have satisfactory levels of compressing characteristics.

Use of water-soluble fillers such as sugar and sugar alcohol which are low in compressibility and increasing the content of the fillers improve the mouth feel of tablets, but on the other hand, increasing the content of such fillers makes the compressing characteristics poor, as described above.

There have been developed no tablets that have not only a satisfactory mouth feel but also excellent compressing characteristics. Tablets have been desired to be developed which overcome the two conflicting problems: improvement in mouth feel of tablets and improvement in compressing characteristics at a stroke, in addition, which are easy to produce and have a good productivity.

### DISCLOSURE OF THE INVENTION

One object of this invention is to provide tablets that have a satisfactory mouth feel and are excellent in compressing characteristics, friability and mechanical strength.

Another object of this invention is to provide orally disintegrating tablets that are easy to produce, have sufficient compressing characteristics, friability and mechanical strength, and can disintegrate rapidly in the oral cavity.

The inventor of this invention has directed tremendous research effort toward the solution of the above described problems and have finally found that the above described problems can be solved by blending a drug formulation with a large amount of water-soluble filler blended thereinto, with a specific amount of a compressing characteristic improving agent that contains a specific amount of moisture. This invention has been accomplished based on such a finding.
1. This invention is tablets produced by blending a powdery or granular mixture including (1) an effective amount of a pharmacologically active substance and (2) 50% by weight or more of a water-soluble filler based on the powdery or granular mixture, with (3) a compressing characteristic improving agent, which contains moisture in an amount equal to or more than the equilibrated moisture content under the conditions of a temperature of 25°C and a humidity of 12% and has an average particle size of 100 µm or less, at a ratio of from 1 to 50% by weight based on the powdery or granular mixture; and compressing the resultant blend.
2. This invention is a process for producing tablets, comprising the steps of: blending a powdery or granular mixture including (1) an effective amount of a pharmacologically active substance and (2) 50% by weight or more of a water-soluble filler based on the powdery or granular mixture, with (3) a compressing characteristic improving agent, which contains moisture in an amount equal to or more than the equilibrated moisture content under the conditions of a temperature of 25°C and a humidity of 12% and has an average particle size of 100 µm or less, at a ratio of from 1 to 50% by weight based on the powdery or granular mixture; and compressing the resultant blend.
3. This invention is an orally disintegrating tablet produced by: blending a powdery or granular mixture including (1) an effective amount of a pharmacologically active substance and (2) 50% by weight or more of a water-soluble filler based on the powdery or granular mixture, with (3) a compressing characteristic improving agent, which contains moisture in an amount equal to or more than the equilibrated moisture content under the conditions of a temperature of 25°C and a humidity of 12% and has an average particle size of 100 µm or less, at a ratio of 1 to 50% by weight based on the powdery or granular mixture; and compressing the resultant blend.
4. This invention is a process for producing an orally disintegrating tablet, including the steps of: blending a powdery or granular mixture comprising (1) an effective amount of a pharmacologically active substance and (2) 50% by weight or more of a water-soluble filler based on the powdery or granular mixture, with (3) a compressing characteristic improving agent, which contains moisture in an amount equal to or more than the equilibrated moisture content under the conditions of a temperature of 25°C and a humidity of 12% and has an average particle size of 100 µm or less, at a ratio of 1 to 50% by weight based on the powdery or granular mixture; and compressing the resultant blend.

### BEST MODE FOR CARRYING OUT THE INVENTION

The tablets in accordance with this invention are obtained by blending a powdery or granular mixture including an effective amount of a pharmacologically active substance and water-soluble filler with a specific compressing characteristic improving agent and compressing the blend.

First, the powdery or granular mixture will be described.

As a pharmacologically active substance, any one of known pharmacologically active substances can be widely used. Such pharmacologically active substances include: for example, common pharmacologically active substances which are blended into various formulations such as formulations for respiratory organ, digestive organ, cardiovascular organ, central nervous system and peripheral nervous system; antibiotics; chemotherapeutics; antineoplastics; platelet aggregation inhibitor; antiallergics; and vitamins. Specific examples of the above formulations include theophylline, cilostazol, grepafloxacin, carteolol, procaterol, rebamipide, aripiprazole and the like. The powdery or granular mixture is to contain an effective amount of a pharmacologically active substance.

As a water-soluble filler, any one of known water-soluble fillers can be widely used. Such fillers include: for example, sugar, sugar alcohol, water-soluble polymer substances and agar.

Specific examples of sugar include: monosaccharides such as glucose, fructose and sucrose; oligosaccharides such as maltose, lactose, saccharose and trehalose; and the like.

Specific examples of sugar alcohols include: mannitol, sorbitol, maltol, erythritol and xylitol.

Specific examples of water-soluble polymers include: polyethylene glycol (macrogol) and polyvinyl alcohol.

These water soluble fillers are used independently or as a mixture of two kinds or more.

Among the above described water-soluble fillers, sugar alcohol is preferably used.

The content of the above described water-soluble fillers in the powdery or granular mixture is usually 50% by weight or more, preferably 60 to 99% by weight, and more preferably 70 to 99% by weight.

The above described powdery or granular mixture may contain, besides a pharmacologically active substance and a water-soluble filler, other additives properly selected depending on the situation. The additives include: for example, perfumes and sweetening agents.

Examples of perfumes include orange extract, orange oil, spearmint oil, peppermint oil, vanilla flavor, lemon oil and 1-menthol. Examples of sweetening agents include aspartame, sodium saccharide, sweet tea, powdered licorice, glycerin, honey and starch syrup.

These additives are used independently or as a mixture of two or more kinds.

Preferably the mixture containing a pharmacologically active substance and a water-soluble filler is in the form of granules. The granular mixture is produced by, for example, mixing a pharmacologically active substance and a water-soluble filler and then granulating the resultant powdery mixture. Granulation can be carried out by any one of known methods such as wet granulation and dry granulation.

In the production of tablets in accordance with this invention, a compressing characteristic improving agent is used which contains moisture in an amount equal to or more than the equilibrated moisture content under the conditions of a temperature of 25°C and a humidity of 12%.

Examples of compressing characteristic improving agents which contain moisture in an amount equal to or more than the equilibrated moisture content under the conditions of a temperature of 25°C and a humidity of 12% and have an average particle size of 100 µm or less are starches, celluloses and polymer substances that meet the above described requirements.

Such starches include: for example, wheat starch, rice starch, corn starch, potato starch, dextrin, pregelatinized starch, partially pregelatinized starch, hydroxypropyl starch, carboxymethyl starch, α-cyclo dextrin, β-cyclo dextrin and pullulan.

Such celluloses include: for example, crystal cellulose, hydroxypropyl cellulose, hydroxypropyl cellulose with low substitution degree, hydroxypropyl methylcellulose, carmellose, carmellose calcium, carmellose sodium and cross carmellose sodium.

Examples of polymer substances include: synthetic polymer substances such as polyvinyl pyrrolidone and polyvinyl alcohol; and natural polymer substances such as agar and gelatin.

Of the above described compressing characteristic improving agents, corn starch, potato starch, crystal cellulose, hydroxypropyl cellulose with low substitution degree and polyvinyl pyrrolidone are preferable.

The equilibrated moisture content under the conditions of a temperature of 25°C and a humidity of 12% varies with respect to the kind of a compressing characteristic improving agent. The equilibrated moisture content under the conditions of a temperature of 25°C and a humidity of 12% is about 5.3% for corn starch, about 1.5% for potato starch, about 4% for partially pregelatinized starch, about 2.3 to 2.5% for crystal cellulose, about 3.0 to 3.1% for hydroxypropyl cellulose with low substitution degree, and about 3.5 to 3.8% for polyvinyl pyrrolidone.

In this invention, the equilibrated moisture content under the conditions of a temperature of 25°C and a humidity of 12% is obtained by putting each compound in a desiccator which contains a saturated solution of lithium chloride to allow the compound to stand at 25°C for 48 hours and then measuring the water content of the compound with a Karl Fischer water content analyzer (trade name: Model KF-06, by Mitsubishi Chemical Industries Ltd.).

An average particle size is about 2 to 32 µm for corn starch, about 70 to 90 µm for potato starch, about 70 µm (mean value) for partially pregelatinized starch, about 17 to 40 µm for crystal cellulose, about 25 to 50 µm for hydroxypropyl cellulose with low substitution degree, and about 30 to 75 µm for polyvinyl pyrrolidone.

These compressing characteristic improving agents are used independently or as a mixture of two or more kinds.

The amount of the compressing characteristic improving agents blended is usually 1% by weight or more, preferably 2 to 50% by weight, and more preferably 5 to 20% by weight per 100% of a powdery or granular mixture that contains a pharmacologically active substance and a water-soluble filler.

The tablet in accordance with this invention is produced by blending a mixture that contains a pharmacologically active substance and a water-soluble filler, with the above described specific compressing characteristic improving agent(s) in a specific amount and then compressing the resultant blend.

In compressing the blend, known compressing means can be widely used. Apparatus used in producing the tablet may be that commonly used in producing conventional oral formulations and no special apparatus is needed.

Blending the above described specific compressing characteristic improving agents contributes to improving the tablet characteristics and thus it eliminates the unfavorableness, such as compressing defect like capping and lack of tablet hardness, when shaping the blend into a tablet. As a result, production of tablets does not require special production apparatus and the tablets of this invention can be produced using tablet producing apparatus commonly in use.

The tablets of this invention have sufficient friability and mechanical strength, thereby being avoidable chipping off the tablets during the operation of packing and unpacking a blister pack (PTP).

The tablets of this invention promptly disintegrate or dissolve in the oral cavity and therefore they are very easily taken by pediatric patients and geriatric patients.

### EXAMPLES

In the following this invention will be described in more detail taking several examples and comparative examples. The details of the compressing characteristic improving agents used herein are as follows. The moisture content of each type of compressing characteristic improving agent was measured with a Karl Fischer water content analyzer.
Corn starch: from Nihon Shokuhin Kako Co., Ltd., 2 to 32 µm in an average particle size, 5.3% in equilibrated moisture content after stored for 48 hours under the conditions of a temperature of 25°C and a humidity of 12%
Potato starch: from Nippon Starch Chemical Co., Ltd., 70 to 90 µm in an average particle size, 1.5% in equilibrated moisture content after stored for 48 hours under the conditions of a temperature of 25°C and a humidity of 12%
AVICEL PH-F20: crystal cellulose, from Asahi Chemical Industry Co., Ltd., 17 µm in an average particle size, 2.5% in equilibrated moisture content after stored for 48 hours under the conditions of a temperature of 25°C and a humidity of 12%
AVICEL PH-301: crystal cellulose, from Asahi Chemical Industry Co., Ltd., 40 µm in an average particle size, 2.3% in equilibrated moisture content after stored for 48 hours under the conditions of a temperature of 25°C and a humidity of 12%
LH-31: hydroxypropyl cellulose with low substitution degree, from Shin-Etsu Chemical Co., Ltd., 25 µm in an average particle size, 3.1% in equilibrated moisture content after stored for 48 hours under the conditions of a temperature of 25°C and a humidity of 12%
LH-11: hydroxypropyl cellulose with low substitution degree, from Shin-Etsu Chemical Co., Ltd., 50 µm in an average particle size, 3.0% in equilibrated moisture content after stored for 48 hours under the conditions of a temperature of 25°C and a humidity of 12%
PVP (XL-10): polyvinyl pyrrolidone, from ISP Japan Ltd., 30 µm in an average particle size, 5.8% in equilibrated moisture content after stored for 48 hours under the conditions of a temperature of 25°C and a humidity of 12%
PVP (XL): polyvinyl pyrrolidone, from ISP Japan Ltd., 75 µm in an average particle size, 3.5% in equilibrated moisture content after stored for 48 hours under the conditions of a temperature of 25°C and a humidity of 12%
FUJICALIN SG: calcium phosphate, from Fuji Chemical Industry Co., Ltd., 113 µm in an average particle size, 0.9% in equilibrated moisture content after stored for 48 hours under the conditions of a temperature of 25°C and a humidity of 12%

The above described types of compressing characteristic improving agents were dry treated (heated at 80°C for 3 hours or more in vacuum) to obtain adjusted agents A. The above described types of compressing characteristic improving agents were wet treated (allowed to stand under the conditions of a temperature of 25°C and a humidity of 60% for 24 hours) to obtain adjusted agents D. The adjusted agents A and the adjusted agents D were mixed to obtain adjusted agents B and adjusted agents C. The moisture contents of these adjusted agents are summarized in Table 1.

**Table 1**

| Improving Agent of (3) | Moisture Content After Adjustment (%) | | | |
|---|---|---|---|---|
| | Adjust Agent A | Adjust Agent B | Adjust Agent C | Adjust Agent D |
| Corn Starch | 3.0 | 6.1 | 9.1 | 13.5 |
| Potato Starch | 0.7 | | | 9.2 |
| AVICEL PH-F20 | 1.7 | 2.7 | 6.2 | 8.3 |
| AVICEL PH-301 | 1.2 | | | 6.7 |
| LH-31 | 1.2 | 4.0 | 7.9 | 10.5 |
| LH-11 | 0.8 | | | 6.5 |
| PVP (XL-10) | 1.7 | | | 18.7 |
| PVP (XL) | 0.9 | 6.1 | 11.3 | 18.5 |
| FUJICALIN SG | 0.6 | | | 2.5 |

### Compressing Test 1

Sugar alcohol-based granules (formulations 1 to 3) shown in Table 2 below were prepared.
Granulation was carried out for each type of granule by mixed fluid bed granulation using Multiplex MP-01 (manufactured by Powrex).

**Table 2**

| | Formulation 1 | Formulation 2 | Formulation 3 |
|---|---|---|---|
| Erythritol (mg) | 147 | | |
| Mannitol (mg) | | 147 | |
| Xylitol (mg) | | | 147 |
| HPC-L (mg) | 3 | 3 | 3 |
| Total (mg) | 150 | 150 | 150 |
| HPC-L: from Nipon Soda Co., Ltd. | | | |

The granule sizes of formulations 1, 2 and 3 were 133 µm, 102 µm and 299 µm, respectively.

0.5% by weight of lubricant (magnesium stearate, from Taihei Chemical Industrial Co., Ltd.) was added to each of the formulations 1 to 3 and each lubricant-added formulation was compressed at a compressing pressure of 1000 kg using a tablet machine (12 HUK, manufactured by Kikusui Seisakusho Co., Ltd.). A die with punches having a diameter of 8 mm and convex with bevel was used in compressing.

The compressing characteristics (whether or not capping occurs and whether or not pressure increases when pulling out the punch or abnormal noise is emitted, at the time of compressing) were examined. When compressing the mixture of the granules of formulation 1 and magnesium stearate, occurrence of capping was observed. When compressing the mixture of formulation 2 or formulation 3 and magnesium stearate, pressure increase in pulling out the punch and emission of abnormal noise were observed. These results show that any one of the above mixtures is poor in compressing characteristics.

Ten percents (10%) by weight of each of the adjusted compressing characteristic improving agents A to D were added to each of the formulations 1 to 3. Further, 0.5% by weight of lubricant (magnesium stearate, from Taihei Chemical Industrial Co., Ltd.) was added to the granule mixtures to which the adjusted agents had been added. The resultant mixtures were compressed at a compressing pressure of 1000 kg using a tablet machine (12 HUK, manufactured by Kikusui Seisakusho Co., Ltd.). A die with punches having a diameter of 8 mm and convex with bevel was used in compressing.

The compressing characteristics (whether or not capping occurs and whether or not pressure increases when pulling out the punch or abnormal noise is emitted, at the time of compressing) were examined. The compressing characteristics were classified in 3 grades:
× (poor): no improvement was observed in compressing defects (occurrence of capping, pressure increase when pulling out the punch or emission of abnormal noise) or the compressing defects were worsened
Δ (good): improvement was observed in compressing defects (occurrence of capping, pressure increase when pulling out the punch or emission of abnormal noise) or almost no compressing defects were observed
○ (excellent): no compressing defects (occurrence of capping, pressure increase when pulling out the punch or emission of abnormal noise) were observed,
and judged for the granules of formulations 1 to 3 compared with the mixtures to which 0.5% by weight of lubricant (magnesium stearate, from Taihei Chemical Industrial Co., Ltd.) alone had been added. The results are shown in Table 3 and Table 4.

**Table 3**

| Formulation 3 | | |
|---|---|---|
| Improving Agent of (3) | Adjusted Improving Agents | Compressing Characteristics |
| Corn Starch | A | × |
| | B | Δ |
| | C | ○ |
| | D | ○ |
| Potato Starch | A | × |
| | D | Δ |
| AVICEL PH-F20 | A | × |
| | B | ○ |
| | D | ○ |
| AVICEL PH-301 | A | × |
| | D | ○ |
| LH-31 | A | × |
| | B | ○ |
| | D | ○ |
| LH-11 | A | × |
| | D | ○ |
| PVP (XL-10) | A | × |
| | D | ○ |
| PVP (XL) | A | × |
| | B | Δ |
| | C | ○ |
| | D | ○ |
| FUJICALIN SG | A | × |
| | D | × |

**Table 4**

| Improving Agent of (3) | Adjusted Improving Agents | Formulation 1 | Formulation 2 |
|---|---|---|---|
| | | Compressing Characteristics | Compressing Characteristics |
| Corn Starch | A | × | × |
| | B | Δ | Δ |
| | C | Δ | ○ |
| | D | ○ | ○ |
| PVP (XL) | A | × | × |
| | B | Δ | Δ |
| | D | ○ | ○ |
| FUJICALIN SG | A | × | × |
| | D | × | × |

Table 3 and Table 4 indicate that for FUJICALIN SG, which contains moisture in an amount equal to or more than the equilibrated moisture content after it is stored under the conditions of a temperature of 25°C and a humidity of 12% for 48 hours but has an average particle size more than 100 µm, compressing characteristics cannot be improved.

### Compressing Test 2

Sugar alcohol-based granules of a formulation shown below were prepared. Granulation was carried out by stirring fluidized-bed granulation using Multiplex MP-01 (manufactured by Powrex).

| Formulation 4 of Granules | |
|---|---|
| Erythritol | 117 parts by weight |
| Corn Starch | 30 parts by weight |
| HPC-L | 3 parts by weight |

The granule size of formulation 4 was 161 µm.

0.5% by weight of lubricant (magnesium stearate, from Taihei Chemical Industrial Co., Ltd.) was added to the granules of the formulation 4 and compressed at a compressing pressure of 1000 kg using a tablet machine (12 HUK, manufactured by Kikusui Seisakusho Co., Ltd.). A die with punches having a diameter of 8 mm and convex with bevel was used in compressing.

The compressing characteristics (whether or not capping occurs and whether or not pressure increases when pulling out the upper punch or abnormal noise is emitted, at the time of compressing) were examined. When compressing the mixture of the granules of the above formulation and magnesium stearate, occurrence of capping was observed.

Ten percents (10%) by weight of corn starch (each of the adjusted compressing characteristic improving agents A to D) were added to the granules of the formulation 4. 0.5% by weight of lubricant (magnesium stearate, from Taihei Chemical Industrial Co., Ltd.) was further added to the granule mixtures to which the adjusted agents A to D had been added. The resultant mixtures were compressed at a compressing pressure of 1000 kg using a tablet machine (12 HUK, manufactured by Kikusui Seisakusho Co., Ltd.). A die with punches having a diameter of 8 mm and convex with bevel was used in compressing.

The compressing characteristics (whether or not capping occurs and whether or not pressure increases when pulling out the upper punch or abnormal noise is emitted, at the time of compressing) were examined and judged in the same manner as in Compressing Test 1.

The mixture of the granules of the formulation 4 and the adjusted improving agent A was judged to be × (poor), the mixture of the granules of the formulation 4 and the adjusted improving agent B was judged to be Δ (good), the mixture of the granules of the formulation 4 and the adjusted improving agent C was judged to be ○ (excellent), and the mixture of the granules of the formulation 4 and the adjusted improving agent D was also judged to be ○ (excellent).

### Compressing Test 3

5 percents by weight or 10% by weight of corn starch (the adjusted improving agent D) were added to the granules of the formulation 4 (compressing test 2). 0.5% by weight of lubricant (magnesium stearate, from Taihei Chemical Industrial Co., Ltd.) was further added to the granule mixtures to which the adjusted improving agent D had been added. The resultant mixtures were compressed at a compressing pressure of 1000 kg using a tablet machine (12 HUK, manufactured by Kikusui Seisakusho Co., Ltd.). A die with punches having a diameter of 8 mm and convex with bevel was used in compressing. For comparison, the granules of the formulation 4 to which no corn starch (the adjusted improving agent D) had been added was compressed at a compressing pressure of 1000 kg.

The average weight (mg), average hardness (kp) and average thickness (mm) were measured for each of the resultant tablets.

The average weight (mg) is the average weight of 10 tablets.

The average hardness (kp) is the value obtained by averaging the hardness of 5 tablets measured using a tablet hardness tester (Model 6D, manufactured by Schleuniger).

The average thickness (mm) is the value obtained by averaging the thickness of 5 tablets measured using a thickness meter (Model G, manufactured by PEACOCK).

Each of the resultant tablets was used for a disintegration test. The disintegration test was carried out in accordance with The Japanese Pharmacopoeia (14th revised edition) using a disintegration tester (Model NF-2F, manufactured by Toyama Sangyo Co., Ltd.). The disintegrating time (second) of each tablet in the oral cavity is shown by the average value of 2 tablets.

The compressing characteristics (whether or not capping occurs) of the mixtures were examined and judged in the same manner as in Compressing Test 1.

The results are shown in Table 5.

**Table 5**

| Amount of Improving Agent of (3) Added | 0% | 5% | 10% |
|---|---|---|---|
| Compressing Pressure (kg) | 1000 | 1000 | 1000 |
| Average Weight (mg) | 150.4 | 158.2 | 168.9 |
| Average Hardness (kp) | 3.5 | 5.3 | 5.8 |
| Average Thickness (mm) | 2.96 | 3.06 | 3.22 |
| Disintegrating Time (second) | 22∼28 | 20∼30 | 23∼41 |
| Orally Disintegrating Time (second) | 18 | 19 | 19 |
| Occurrence of Capping | occur | not occur | not occur |

In the granule mixtures to which no corn starch (adjusted improving agent D) had been added, occurrence of capping was observed, whereas in the granule mixtures to which 5% by weight of corn starch (adjusted improving agent D) had been added, occurrence of capping could be completely prevented. The increase in tablet hardness with increase in amount of corn starch added was also observed. The granule mixtures to which no corn starch (adjusted improving agent D) had been added and those to which 5% by weight of corn starch (adjusted improving agent D) had been added are almost equal in disintegrating characteristics. While in the disintegrating test addition of 10% by weight of corn starch (adjusted improving agent D) caused the disintegrating characteristics to deteriorate a little, in the oral cavity almost no difference was observed in disintegrating characteristics between the granule mixtures to which 10% by weight of corn starch (adjusted improving agent D) had been added and those to which no corn starch or 5% by weight of the same had been added.

### Compressing Test 4

Tablets were produced from the formulation shown in Table 6 below in the same manner as in Compressing Test 1.

The average weight (mg), average thickness (mm), average hardness (kp) and disintegrating time (second) of the resultant tablets were measured in the same manner as in Compressing Test 3.

The friability (%) was determined by subjecting the resultant tablets (20 tablets) to friability test for 15 minutes using an friability tester (Model TFT-120, manufactured by Toyama Sangyo Co.) at 25 rpm.

The compressing characteristics (whether or not capping occurs) were examined and judged in the same manner as in Compressing Test 1.

The results are shown in Table 6.

**Table 6**

| | Formulation 5 | Formulation 6 | Formulation 7 |
|---|---|---|---|
| Aripiprazole (mg) | 15 | 15 | 15 |
| Erythritol (mg) | 102 | 102 | 102 |
| Corn Starch (mg) | 30 | 30 | 30 |
| HPC-L (mg) | 3 | 3 | 3 |
| (3) Improving Agents Corn Starch (mg) | 15 | | |
| PCS (mg) | | 7.5 | |
| PVP (XL) (mg) | | | 7.5 |
| Magnesium Stearate (mg) | 0.8 | 0.8 | 0.8 |
| Total (mg) | 165.8 | 158.3 | 158.3 |
| Compressing Pressure (kg) | 600 | 600 | 600 |
| Average Weight (mg) | 166.3 | 157.8 | 157.4 |
| Average Thickness (mm) | 3.25 | 3.14 | 3.18 |
| Average Hardness (kp) | 3.3 | 3.4 | 3.7 |
| Disintegrating time (water) (second) | 24∼29 | 25∼30 | 15∼20 |
| Compressing Characteristics | good | good | good |
| Moisture content of corn starch: 13.5% | | | |
| Moisture content of PCS: 12.3% | | | |
| Moisture content of PVP (XL): 18.5% | | | |

### Compressing Test 5

Tablets were produced from the formulation shown in Table 7 below in the same manner as in Compressing Test 1.

The average weight (mg), average thickness (mm), average hardness (kp) and disintegrating time (second) of the resultant tablets were measured in the same manner as in Compressing Test 3.

The friability (%) was determined in the same manner as in Compressing Test 4.

The compressing characteristics (whether or not capping occurs) were examined and judged in the same manner as in Compressing Test 1.

The results are shown in Table 7.

**Table 7**

| | Formulation 8 | Formulation 9 | Formulation 10 | Formulation 11 |
|---|---|---|---|---|
| Pharmacologically | 15 | 15 | | |
| Active Substance A (mg) | | | | |
| Pharmacologically | | | | |
| Active Substance B (mg) | | | 15 | 15 |
| Erythritol (mg) | 102 | 102 | 102 | 102 |
| Corn Starch (mg) | 30 | 30 | 30 | 30 |
| HPC-L (mg) | 3 | 3 | 3 | 3 |
| (3) Improving Agents Corn Starch (mg) | 15 | | 15 | |
| PVP (XL) (mg) | | 7.5 | | 7.5 |
| Magnesium Stearate (mg) | 0.8 | 0.8 | 0.8 | 0.8 |
| Total (mg) | 165.8 | 158.3 | 165.8 | 158.3 |
| Compressing Pressure (kg) | 800 | 800 | 600 | 600 |
| Average Weight (mg) | 165.5 | 158.4 | 168.1 | 157.6 |
| Average Thickness (mm) | 3.18 | 3.14 | 3.33 | 3.23 |
| Average Hardness (kp) | 3.7 | 3.9 | 3.7 | 3.4 |
| Disintegrating time (water) (second) | 24∼35 | 21∼23 | 35∼40 | 25∼28 |
| Friability (%) | 0.7 | 0.3 | 1.4 | 1.0 |
| Compressing Characteristics | good | good | good | good |
| Moisture content of corn starch: 13.5% | | | | |
| Moisture content of PVP (XL): 18.5% | | | | |

The pharmacologically active substance A in Table 7 was 1-[3-{4-(3-chlorophenyl)-1-piperazinyl}propyl]-5-methoxy-3,4-dihydro-2(1H)-quinoline monomethanesulfonate. The pharmacologically active substance B was (5R)-2-[1-{2-chloro-4-(1-pyrrolidinyl)benzoyl}-2,3,4,5-tetrahydro-1H-1-benzazepine-5-yl]-N-isopropyl acetamide.

### Compressing Test 6

Tablets were produced from the formulation shown in Table 8 below in the same manner as in Compressing Test 1.

The average weight (mg), average thickness (mm), average hardness (kp) and disintegrating time (second) of the resultant tablets were measured in the same manner as in Compressing Test 3.

The compressing characteristics (whether or not capping occurs) were examined and judged in the same manner as in Compressing Test 1.

The results are shown in Table 8.

**Table 8**

| | Formulation 12 | Formulation 13 |
|---|---|---|
| Cilostazol (mg) | 50 | 50 |
| Erythritol (mg) | 146 | 48 |
| HPC-L (mg) | 4 | 2 |
| (3) Improving Agents PVP (XL) (mg) | 10 | 5 |
| Magnesium Stearate (mg) | 1.1 | 0.5 |
| Total (mg) | 211.1 | 105.5 |

| Shape of Die with Punches | 10.5 mm in diameter, convex with bevel | 7 mm in diameter, Sugar-Coated R (5.5R) |
|---|---|---|
| Compressing Pressure (kg) | 600 | 300 |
| Average Weight (mg) | 212.9 | 105.3 |
| Average Thickness (mm) | 3.16 | 3.58 |
| Average Hardness (kp) | 4.3 | 5.6 |
| Disintegrating time (water) (second) | 15∼20 | 20∼25 |
| Compressing Characteristics | good | good |
| Moisture content of PVP (XL): 18.5% | | |

## Claims

1. Tablets produced by blending a powdery or granular mixture comprising (1) an effective amount of a pharmacologically active substance and (2) 50% by weight or more of a water-soluble filler based on the powdery or granular mixture with (3) a compressing characteristic improving agent, which contains moisture in an amount equal to or more than the equilibrated moisture content under the conditions of a temperature of 25°C and a humidity of 12% and has an average particle size of 100 µm or less, at a ratio of from 1 to 50% by weight based on the a powdery or granular mixture; and compressing the resultant blend.

2. A process for producing tablets, **characterized in that** the process comprises the steps of: blending a powdery or granular mixture comprising (1) an effective amount of a pharmacologically active substance and (2) 50% by weight or more of a water-soluble filler based on the powdery or granular mixture with (3) a compressing characteristic improving agent, which contains moisture in an amount equal to or more than the equilibrated moisture content under the conditions of a temperature of 25°C and a humidity of 12% and has an average particle size of 100 µm or less, at a ratio of from 1 to 50% by weight based on the powdery or granular mixture; and compressing the resultant blend.
